Europäisches Patentamt

European Patent Office

Office européen des brevets

(11) Publication number: **0 345 780 B1**

(19)

(12) # EUROPEAN PATENT SPECIFICATION

(45) Date of publication of patent specification: **10.11.93**   (51) Int. Cl.⁵: **A61M 1/18**, B01D 61/00

(21) Application number: **89110390.5**

(22) Date of filing: **08.06.89**

(54) **Hollow fiber type blood purification apparatus.**

(30) Priority: **09.06.88 JP 142621/88**

(43) Date of publication of application:
**13.12.89 Bulletin 89/50**

(45) Publication of the grant of the patent:
**10.11.93 Bulletin 93/45**

(84) Designated Contracting States:
**DE ES FR GB IT SE**

(56) References cited:
**EP-A- 0 249 189
US-A- 4 024 059
US-A- 4 402 940**

(73) Proprietor: **NIKKISO CO., LTD.
No. 43-2, Ebisu 3-chome
Shibuya-ku Tokyo 150(JP)**

(72) Inventor: **Chiba, Toshiaki
43-2, Ebisu 3-chome
Shibuya-ku Tokyo(JP)**

(74) Representative: **TER MEER - MÜLLER - STEIN-
MEISTER & PARTNER
Mauerkircherstrasse 45
D-81679 München (DE)**

EP 0 345 780 B1

EP 0 345 780 B1

**Description**

BACKGROUND OF THE INVENTION

(1) Field of the Invention

The present invention relates to a blood purification apparatus and more particularly to a blood purification apparatus which can effectively purify blood without causing contamination of blood with foreign substances or pyrogen present in dialysis solution by preventing back filtration during hemodialysis.

(2) Description of the Related Art

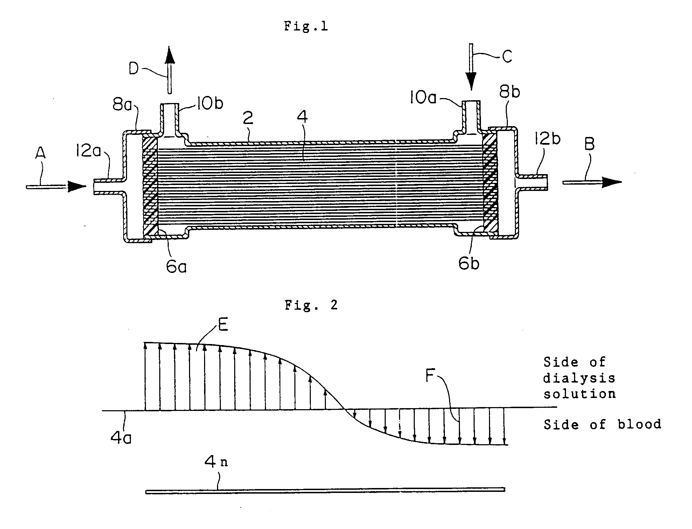

Fig.1 illustrates a blood purification apparatus which dialyzes and filtrates blood to purify it.

In Fig.1, a cylindrical housing is indicated by (2), a hollow fiber bundle packed in this housing (2) is indicated by (4), a pair of supports which support both ends of the hollow fiber bundle (4) at both ends of housing (2) are indicated by (6a) and (6b) and caps which cover both ends of housing (2) are indicated by (8a) and (8b).

Introduction opening (10a) for introduction of a dialysis solution into this housing (2) is formed projecting from side wall of one end portion of housing (2) and discharge opening (10b) for discharging a dialysis solution from housing (2) is formed projecting from side wall of another end portion.

Supports (6) are made of a synthetic resin. The supports (6) are in the form of a thick disk having a diameter nearly the same as the inner diameter of openings at both ends of housing (2). The supports (6) integrally fix both ends of hollow fiber bundle (4) so that the hollow fiber bundle (4) is not unbound. Openings at both ends of the hollow fibers are exposed at outer surface of supports (6).

Introduction opening (12a) for introducing blood into this blood purification apparatus is formed projecting from cap (8a) and discharge opening (12b) for discharging blood from the apparatus is formed projecting from another cap (8b).

Blood is introduced, as shown by arrow A, from introduction opening (12a) into a space in cap (8a), namely, into a space formed by cap (8a) and support (6a), then is passed through respective hollow fibers (4n) of hollow fiber bundle (4), enters into a space in cap (8b), namely, a space formed by support (6b) and cap (8b) and is discharged from discharge opening (12b) as shown by arrow B. Dialysis solution is introduced, as shown by arrow C, into housing (2) from introduction opening (10a), flows in the direction opposite to the flow of blood to be dialyzed through outside of hollow fibers (4n) and is discharged from dialysis solution discharge opening (10b) as shown by arrow D. Waste materials in the blood in the hollow of hollow fibers (4n) are dialyzed through the wall of hollow fibers (4n) and migrate into the dialysis solution of the outside.

In general, blood purification apparatuses aim at removal of substances having a molecular weight of 100-200 as waste materials. Therefore, pores having such a size as being able to pass these substances, for example, 30-40 Å are formed through the wall of the hollow fibers used in the blood purification apparatus. When hemodialysis is carried out for a long period of time using such blood purification apparatus, substances which cannot be removed by such hollow fibers are retained in the blood. As a result, the patients are sometimes attacked with complications such as dialysis amyloidosis.

One of substances to cause this dialysis amyloidosis is considered $\beta_2$ microglobulin. This $\beta_2$ microglobulin is a substance having a high molecular weight of 11,800 and so cannot be removed by the conventional blood purification apparatuses provided with hollow fibers having pores of about 30-40 Å.

Therefore, recently, there have been developed hollow fibers having pores of greater than 50-60 Å as those comprising a high flux membrane having larger pores.

However, when blood is purified by using such hollow fibers (hereinafter, hollow fibers may be sometimes called "membrane") of high flux, degree of removal of water (which is evaluated in terms of ultrafiltration rate and which is referred to as "UFR" hereinafter) also increases in proportion to the pore size. For example, ordinary membrane has a UFR of 10 ml/hr•mmHg•m$^2$ or less while high flux membrane has a UFR of higher than 10 ml/hr•mmHg•m$^2$. Thus, with increase in performace to remove high molecular weight substances, UFR also increases (in some case, as high as 20 ml/hr•mmHg•m$^2$ or higher).

In hemodialisis therapy for purification of the blood, general amount of water to be removed from dialytic patients must be at most about 1-4 liters for one treatment. Besides, if water in such amount is removed outside the body in a short time, reduction in blood pressure occurs and the patients fall into a critical condition. Therefore, water must be gradually removed from the blood of patients over a period of 4-5 hours. Accordingly, water removing rate is usually 250-1,000 ml/hr.

However, in case of using a blood purification apparatus, the purified blood cannot be returned into the body of patients unless a water removing pressure of 50-100 mmHg is applied as a spontaneous pressure due to venous pressure of the patients and flow path resistance of blood circuit. Therefore, when a blood purification apparatus which applies such spontaneous pressure and has a UFR performance of, for example, at least 20 ml/hr•mmHg m$^2$ is used, water is removed at a rate of 1,000-2,000 ml/hr only by the spontaneous pressure. Recently, in order to solve such problem, a blood purification apparatus having high flux membrane is combined with a controller for water removal.

By combining the controller, it has become possible to easily control amount of water to be removed even if the high flux membrane is used.

However, the controller for water removal has the following defects.

The controller is constructed so that it discharges out of the system a constant amount of dialysis solution delivered from the blood purification apparatus. Therefore, if the high flux membrane is used and amount of water to be removed is controlled by the controller, there occurs back filtration in the hollow fibers in the vicinity of exit of the blood, namely, permeation of the dialysis solution into blood because there is necessarily present a pressure loss of about 30-50 mmHg between the blood introduction side and the blood discharging side at a flow rate of blood of 200 ml/min.

Dialysis solution is normally considered not to be incorporated into blood and so quality of the dialysis solution is not controlled supposing that it may be taken in the body. Therefore, if the dialysis solution is incorporated into the blood owing to the back filtration, there is the possibility of foreign matters or pyrogen penetrating into the body together with the dialysis solution.

As explained above, in the blood purification apparatus provided with a high flux diaphragm, not only the controller of water removal is needed, but also there is the problem that foreign matters or pyrogen from the dialysis solution may incorporate into blood owing to the back filtration.

SUMMARY OF THE INVENTION

The present invention has solved the above problems by enhancing the filtration rate of hollow fibers on the blood introduction side and lowering the filtration rate of the hollow fibers on the blood discharging side in a blood purification apparatus comprising a cylindrical housing in which a hollow fiber bundle is packed.

BRIEF DESCRIPTION OF THE DRAWINGS

Fig.1 illustrates a blood purification apparatus, Fig.2 explains state of dialysis according to a conventional blood purification apparatus and Fig.3 explains the state of dialysis according to the present invention.

DESCRIPTION OF THE PREFERRED EMBODIMENTS

Basic construction of the blood purification apparatus of the present invention is the same as that of the apparatus shown in Fig.1.

However, diameter of pores of hollow fiber (4n) membrane differs in the lengthwise direction of the fiber. That is, the hollow fiber (4n) has pores of greater diameter in the portion of blood introduction side and pores of smaller diameter in the portion of blood discharging side.

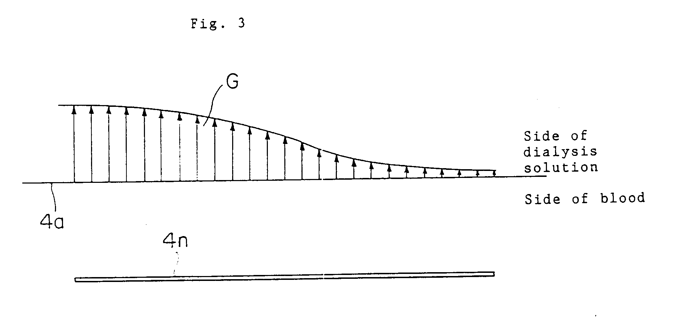

Fig.3 shows the state of dialysis according to the present invention.

In Fig. 3, hollow fibers are shown by (4n) and wall of the hollow fibers is shown by (4a). Dialysis solution side is above wall (4a) of hollow fiber (4n) and blood side is below the wall.

When blood is dialyzed by flowing the blood through the hollow of hollow fiber (4n) and flowing a dialysis solution outside the hollow fiber (4n), waste matters in the blood migrate to the dialysis solution side along the whole hollow fiber (4n) in such a manner that they migrate in a larger amount on the blood introduction side and in a smaller amount on the blood discharging side as indicated by arrow G and no back filtration occurs.

Here, it is preferred that the following sieving coefficient SC of hollow fibers for $\beta_2$ microglobulin (molecular weight: 11,800) is 0.2 or more on the blood introduction side and 0.2 or less on the blood discharging side.

$$SC = \frac{\text{Concentration of solute in ultrafiltrate}}{\text{Concentration of solute in blood}}$$

When the sieving coefficient is less than 0.2 on the blood introduction side, performance for removal of $\beta_2$ microglobulin in a dialyzing time is not satisfactory and when it is more than 0.2 on the blood discharging side, back filtration tends to occur so often.

Furthermore, the hollow fibers used in the present invention preferably have an ultrafiltration rate for water of 5 ml/hr•mmHg•m$^2$ or more on the blood introduction side in addition to the above-mentioned sieving coefficient.

When the ultrafiltration rate is less than 5 ml/hr•mmHg•m$^2$, less amount of high molecular weight substances migrate with migration of water and sometimes it becomes impossible to enhance performance for removal of $\beta_2$ microglobulin.

The hollow fibers used in the present invention include, for example, cellulosic hollow fibers and hollow fibers of synthetic polymers such as polymethyl methacrylate, polyacrylonitrile, polyethylvinyl alcohol, cellulose acetate (diacetate and triacetate), polycarbonate and polysulfone.

The hollow fibers used in the present invention can be produced by any of the following methods.

(1) Lower half of a hollow fiber bundle having pores of large diameter is heat treated. The pores in the portion of the lower half of the fiber bundle contract with heat to become pores of small diameter. The hollow fibers having pores of large diameter can be produced by suitably setting solution concentration, coagulation temperature, etc. for spinning the hollow fibers.

(2) Lower half of a hollow fiber bundle having pores of large diameter is moistened and dried. When dried, cellulosic fibers shrink and the pores in the lower half portion contract to become pores of small diameter. In this case, degree of moisture to be applied cannot be simply specified, but for cellulosic hollow fibers the pores can be easily contracted by treating the fibers with water vapor for about 1 hour and then drying them.

(3) Upper half of cellulosic hollow fiber bundle is immersed in glycerin. This hollow fiber bundle is heat treated. Owing to the presence of glycerin, the pores in the upper half portion are retained as they are even after the heat treatment and the pores in the lower half portion contract to become pores of small diameter.

Example

Lower half of a high flux type cellulosic hollow fiber bundle was immersed in a 40% glycerin solution for 1 hour. Thereafter, this cellulosic hollow fiber bundle was divided into two portions (A) which was immersed in the glycerin solution and (B) which was not immersed. Two modules (dialyzers) of 1.0 m$^2$ were fabricated using the portions (A) and (B), respectively. These modules were respectively subjected to steam sterilization over a period of 30 minutes with heating to 121°C.

These dialyzers were tested in vitro using cytochrome C having a molecular weight of 12,500. As a result, the hollow fibers of the portion (A) corresponding to the blood introduction side had a sieving coefficient of 0.6 and the hollow fibers of the portion (B) corresponding to the blood discharging side had a sieving coefficient of 0.1.

Moreover, the portion (A) corresponding to the blood introduction side had an ultrafiltration rate of 18 ml/hr•mmHg•m$^2$.

A module fabricated using the cellulosic hollow fibers before divided into two portions had a sieving coefficient of 0.56 and an ultrafiltration rate of 9.6 ml/hr•mmHg•m$^2$.

For comparison, the whole of the above high flux type cellulosic hollow fiber bundle was immersed in a 40% glycerin solution and then subjected to steam sterilization under the same conditions as above and then a module of 1.0 m$^2$ was fabricated therefrom. Sieving coefficient of the hollow fibers in this module was 0.6 on the blood introduction side and blood discharging side and ultrafiltration rate was 18 ml/hr•mmHg•m$^2$.

As is clear from the above results, ultrafiltration rate was able to be reduced to nearly half without substantially no reduction of sieving coefficient for cytochrome-C.

The present invention having the above-mentioned construction can exhibit the following effects.

First, there occurs no incorporation of dialysis solution into blood (back filtration) during dialysis. That is, since UFR can be reduced through the whole module, there is a little possibility of back filtration even if amount of water to be removed is set at a low level and besides, substantially no decrease is seen in performance for removal of $\beta_2$ microglobulin.

Furthermore, controller for water removal can be omitted depending on kind of membrane and dialyzing amount. That is, $\beta_2$ microglobulin can be effectively removed with reduction in UFR without using the controller for water removal.

**Claims**

1. A blood purification apparatus comprising a cylindrical housing (2) in which a bundle (n) of hollow fibers is packed characterised in that the hollow fibers (4n) on the blood introduction side have a higher filtration rate and those on the blood discharging side have a lower filtration rate.

2. A blood purification apparatus according to claim 1 characterised in that the following sieving coefficient (SC) of the hollow fibers for $\beta_2$ microglobulin is 0.2 or higher on the blood intriduction side and 0.2 or lower on the blood discharging side:

$$SC = \frac{Concentration\ of\ solute\ in\ ultrafiltrate}{Concentration\ of\ solute\ in\ blood}$$

3. A blood purification apparatus according to claim 1 characterised in that the hollow fibers (4n) on the blood introduction side have an ultrafiltration rate of 5 ml/hr•mmHg•m$^2$ or higher for water.

4. A blood purification apparatus according to claim 1 characterised in that the hollow fiber membrane has pores of greater diameter in the portion of blood introduction side and pores of smaller diameter in the portion of blood discharging side.

**Patentansprüche**

1. Blutreinigungsvorrichtung, umfassend ein zylindrisches Gehäuse (2) mit einer Packung aus einem Bündel (n) aus Hohlfasern, **dadurch gekennzeichnet**, daß die Hohlfasern (4n) auf der Blutzufuhrseite eine höhere Filtrationsgeschwindigkeit und diejenigen auf der Blutentnahmeseite eine niedrigere Filtrationsgeschwindigkeit aufweisen.

2. Blutreinigungsvorrichtung nach Anspruch 1, **dadurch gekennzeichnet**, daß der nachfolgende Siebkoeffizient (SC) der Hohlfasern für $\beta_2$-Mikroglobulin auf der Blutzufuhrseite 0,2 oder darüber und 0,2 oder darunter auf der Blutentnahmeseite beträgt:

$$SC = \frac{Konzentration\ an\ Gelöstem\ im\ Ultrafiltrat}{Konzentration\ an\ Gelöstem\ im\ Blut}$$

3. Blutreinigungsvorrichtung nach Anspruch 1, **dadurch gekennzeichnet**, daß die Hohlfasern (4n) auf der Blutzufuhrseite für Wasser eine Ultrafiltrationsgeschwindigkeit von 5 ml/h•mmHg•m$^2$ oder darüber besitzen.

4. Blutreinigungsvorrichtung nach Anspruch 1, **dadurch gekennzeichnet**, daß die Hohlfasermembran im Bereich der Blutzufuhrseite Poren mit größerem Durchmesser und im Bereich der Blutentnahmeseite Poren mit kleinerem Durchmesser aufweist.

**Revendications**

1.  Appareil de purification du sang comprenant une enveloppe cylindrique (2) dans laquelle est enfermé un faisceau (4) de fibres creuses, caractérisé en ce que les fibres creuses (4n) situées sur le côté d'introduction du sang ont un plus haut taux de filtration et que celles situées sur le côté de sortie du sang ont un taux de filtration plus bas.

2.  Appareil de purification selon la revendication 1, caractérisé en ce que le coefficient de filtration (SC) (défini ci-dessous) des fibres creuses pour la $\beta_2$ microglobuline (poids moléculaire : 11 800) est de 0,2 ou plus sur le côté d'introduction du sang et de 0,2 ou moins sur le côté de sortie du sang.

$$SC = \frac{\text{concentration du soluté dans l'ultrafiltrat}}{\text{concentration du soluté dans le sang.}}$$

3.  Appareil de purification du sang selon la revendication 1, caractérisé en ce que les fibres creuses (4) situées sur le côté d'introduction du sang ont un taux d'ultraliltration de 5 ml/h.mmHg.m$^2$ ou plus pour l'eau.

4.  Appareil de purification du sang selon la revendication 1, caractérisé en ce que la membrane composée des libres creuses a des pores de plus grand diamètre dans la partie située sur le côté d'introduction du sang et des pores de plus petit diamètre dans la partie située sur le côté de sortie du sang.

Fig.1

Fig. 2

Side of
dialysis
solution

Side of blood

Fig. 3

G

Side of
dialysis
solution

Side of blood

4a

4n